# EUROPEAN PATENT SPECIFICATION

(11) **EP 3 372 356 B1**
(45) Date of publication and mention of the grant of the patent: **06.05.2020**
(21) Application number: 17159352.8
(22) Date of filing: 06.03.2017
(51) Int. Cl.: B25J 9/16, A61B 8/08, A61B 34/20, A61B 34/30

(54) **SYSTEM AND METHOD FOR MOTION CAPTURE AND CONTROLLING A ROBOTIC TOOL**
SYSTEM UND VERFAHREN ZUR BEWEGUNGSERFASSUNG UND STEUERUNG EINES ROBOTERWERKZEUGS
SYSTÈME ET PROCÉDÉ DE CAPTURE DE MOUVEMENT ET DE COMMANDE D'UN OUTIL ROBOTIQUE

(43) Date of publication of application: 12.09.2018
(73) Proprietor: Siemens Healthcare GmbH, 91052 Erlangen (DE)
(72) Inventor: Grasser, Frank, 91330 Eggolsheim (DE); Mönnich, Holger, 86316 Friedberg (DE)

(56) References cited:
- DE-A1-102015 204 867
- US-A1- 2011 218 423
- US-B1- 6 322 567
- SHELTEN G YUEN ET AL: "Robotic motion compensation for beating intracardiac surgery", CONTROL, AUTOMATION, ROBOTICS AND VISION, 2008. ICARCV 2008. 10TH INTERNATIONAL CONFERENCE ON, IEEE, PISCATAWAY, NJ, USA, 17 December 2008 (2008-12-17), pages 617-622, XP031433657, ISBN: 978-1-4244-2286-9
- SHELTEN G YUEN ET AL: "3D Ultrasound-Guided Motion Compensation System for Beating Heart Mitral Valve Repair", 6 September 2008 (2008-09-06), MEDICAL IMAGE COMPUTING AND COMPUTER-ASSISTED INTERVENTION - MICCAI 2008; [LECTURE NOTES IN COMPUTER SCIENCE], SPRINGER BERLIN HEIDELBERG, BERLIN, HEIDELBERG, PAGE(S) 711 - 719, XP019105094, ISBN: 978-3-540-85987-1 * the whole document *

## Description

The present invention relates to a system and a method for motion capture and for controlling a robotic medical tool.

For precise manipulation of an object or interaction with an object using a robotic tool it is necessary to control the medical tool with equal precision. Known systems and methods in this field make use of external optical tracking systems comprising a camera and physical markers. The markers are physically attached to the object and may then be tracked with the camera via direct line of sight and can thus be used to track the motion or movement of the object. The use of physical markers as well as the requirement of a direct line of sight between the markers and the camera are often limiting and undesirable.

US 6,322,567 B1 discloses a method of tracking and compensating for bone motion when operating a bone with a surgical robotic arm. Therein, the movements of the bone are tracked with a six degree of freedom position sensor. A registration of the robotic arm to the bone is then updated as the bone moves.

The paper "Robotic Motion Compensation for Beating Intracardiac Surgery" by Shelten G. Yuen et al. for the 2008 10th International Conference on Control, Automation, Robotics and Vision is concerned with the concept of using a one-degree-of-freedom motion compensation system to synchronize instruments with tissue motions that are approximately one-dimensional. This enables the use of 3D ultrasound for both imaging and tracking while avoiding typical delays described in previous work.

US 2011/218423 A1 discloses an apparatus and the corresponding method for combining imaging information gathered by an imaging system including a robot arm that controls the positioning of an ultrasound transducer mounted on the end effector of the robot, and also comprising a CT scan device.

It is an objective of the present invention to facilitate improved control of a robotic tool and an improved workflow.

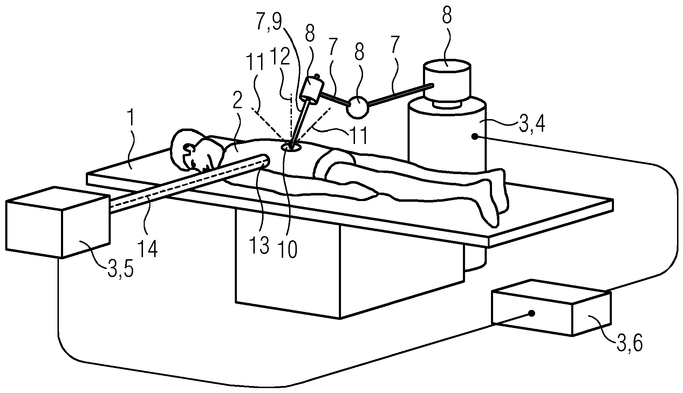

This objective is achieved by a system and a method having the features of the respective independent claims. Advantageous embodiments with expedient developments of the present invention are indicated in the other patent claims as well as in the following description and the drawing.

A system according to the present invention comprises a robotic medical tool, a motion capture device and a control unit. The motion capture device is configured to capture a motion or movement of an object, while the control unit is configured to adjust - and thus control - the robotic tool in dependence of the captured motion or movement. Therein, the motion capture device is an ultrasound device.

For the present invention and specification no distinction shall be made between the terms "motion" and "movement". It is to be understood however, that these terms may refer to a motion of the whole object or a motion of only a part of the object.

In terms of the present invention a robotic tool is to be understood as a device or machine capable of carrying out a complex series of actions automatically. These actions may include movement and positioning of the robotic tool or part thereof in three dimensions, mechanically guiding other devices or an operator, manipulation of and/or interaction with the tracked object, and others. Advantageously, the Robotic tool may be computer programmable so as to allow for specifying in advance the actions to carry out.

The robotic tool may also be described as a medical robot. As such, the robotic tool comprises a tool head which is or comprises the part of the robotic tool that is used to manipulate the object or interact with the object. The tool head may advantageously be changeable to allow for greater flexibility and use cases of the robotic tool. Examples of tool heads may include probes and drills.

The control unit is connected to the robotic tool as well as to the ultrasound device via respective data links. These data links are used to send data representing the captured motion from the ultrasound device to the control unit on the one hand and to send corresponding control signals or commands from the control unit to the robotic tool. The data links may also be bidirectional to enable a two-way exchange of data. This can for example enable a feedback control system. It can also be advantageous for the control unit to be configured to control the ultrasound device, for example to automatically follow a movement of the robotic tool. This advantageously makes it possible to always and automatically capture the motion of precisely the specific part of the object closest to the robotic tool and/or containing a point or area of interaction between the robotic tool and the object. This in turn allows for an especially precise adjustment of the robotic tool relative to the object. The control unit may comprise at least one microcontroller, microprocessor, storage medium, and/or interface to carry out the described actions.

The ultrasound device may comprise an ultrasound head or probe. This ultrasound head may be placed in contact with the object to be tracked. As an example and without limiting the present invention thereto, an especially advantageous use case for the present invention is the tracking of movements of a patient for example during spine surgery. In this use it is especially important to precisely capture and track any patient movements and to control or adjust the robotic tool accordingly to compensate the patient movements. Using the ultrasound device it is for example possible to detect bone material of the patient - for example the spine or a specific vertebra - and hence capture and track any movement of said bone or the patient. This captured and/or tracked movement - or corresponding data - is then processed to generate corresponding control signals for the robotic tool. Through these control signals the robotic tool can be kept in an intended position relative to the patient or, more specifically, relative to the individual bone detected by the ultrasound device.

The ultrasound device may be operated at different ultrasound frequencies. Different frequencies may be suited for capturing and/or tracking different objects and/or different motions and may thus be selected case by case. As an example, an ultrasound device using frequencies between 2 and 15 MHz may provide a lateral resolution between 3 and 0.4 mm and an axial resolution between 0.8 and 0.15 mm.

The present invention can advantageously forgo any additional markers on the object and thereby can for example obviate the need for additional incisions in the patient or other object, especially for minimally invasive procedures. This avoids unnecessary injury of a patient or damage to an object and simultaneously improves a workflow. By not relying on an optical camera system to capture and track the motion of the object the present invention advantageously also avoids occlusion problems where the motion of the object cannot be captured due to another object or person blocking direct line of sight between the camera and the tracked object or marker. The present invention therefore provides improved flexibility and reliability in capturing and/or tracking the motion of the object.

The use cases for the present invention are not limited to patients as objects or medical procedures. It can also be applied to manipulations of any object the motion of which can be detected with the ultrasound device. This is especially the case for objects that are at least partially penetrable by ultrasound and have areas or parts of different density which thus respond different to ultrasound.

In an advantageous refinement of the present invention the system is configured to determine a main plane and/or direction of the motion. Here, the ultrasound device is arranged to capture the motion of the object at least in that plane and/or direction. The system and the ultrasound device may, in other words, be arranged to capture the directional motion of the object with for example the biggest deflection, amplitude, and/or path length. As an example, a vertebra of a patient may substantially only move back and forth in one direction due to a respiratory movement of the patient. This plane or direction may be determined by an operator or it may be determined automatically, in particular it may be determined dynamically during operation of the system. To determine the plane or direction automatically, the object may be scanned or captured from different sides or angles, i.e. the ultrasound device may be set to observe the object from different points of view. If for example the object follows a periodic trajectory, the main plane or direction of movement may be determined by observing the object from different angles over a period of time on the order of the periodic time. Capturing the motion of the object in the main plane or direction of movement can advantageously allow for sufficient capturing precision even with a very simple ultrasound device. The object may for example be secured in such a way that its movement is limited to only one plane or direction. It may also be the case that only one plane or direction of movement is important or relevant to the outcome or success of the procedure performed by or with the robotic tool.

In an advantageous refinement of the present invention the motion capture device is configured to capture the motion of the object in three dimensions. It is, in other words, provided that the motion of the object may be captured regardless of the direction of movement. This allows for precise adjustment of the robotic tool and can increase the flexibility and applicability of the present invention, since it may be used in scenarios where the object and/or the robotic tool moves in a complex pattern. To capture the three-dimensional motion of the object the ultrasound device may comprise a 3D-ultrasound head or probe.

In an advantageous refinement of the present invention the ultrasound device comprises at least two ultrasound heads. The two ultrasound heads or probes may be arranged in the same housing to effectively form a compact and simple to use 3D-ultrasound device. The two ultrasound heads may also be spaced apart from each other and may in particular be attached to different mounts or bearings to allow independent positioning of two ultrasound heads. This advantageously enables each ultrasound head to be built very small and compact while at the same time making it possible to capture the motion of the object in three dimension - at least under most real conditions. The two ultrasound heads can be arranged or positioned very flexibly, which enable the use of the system in a wide variety of situations. The two ultrasound heads may also be used to capture the motions of different parts of the object, which can enable a more sophisticated and precise adjustment of the robotic tool relative to the object.

In an advantageous refinement of the present invention the system is configured to scan the object and to then register the medical tool to a coordinate system of the scan. It is, in other words, provided that a frame of reference and a position of the object in that frame of reference is established and the robotic tool is aligned or synchronized to that frame of reference. This advantageously allows for absolute positioning of different components such as the robotic tool, the ultrasound device, and others with respect to the object independently of each other. If for example the robotic tool is moved out of a coverage range or coverage area of the ultrasound device the alignment and thus knowledge about the precise relative positioning is not automatically lost. To support this functionality any motion of the robotic tool and/or the ultrasound device can preferably be tracked. This may for example be achieved through a motorised bearing, mount, and/or control of the robotic tool and/or the ultrasound respectively.

In an advantageous refinement of the present invention the system is configured to scan the object using the ultrasound device. The ultrasound device may, in other words, be moved along or around the object to scan it and establish a frame of reference and a position of the object within that frame of reference. This advantageously allows for registering the different system components that can be moved independently of each other to one another without the need for any further equipment.

In an advantageous refinement of the present invention the system is configured to scan the object with an X-ray apparatus. Through the scan a frame of reference and a position of the object within that frame of reference can be established. The robotic tool and the X-ray apparatus may therefore be registered to one another. The X-ray apparatus may advantageously provide more precise and/or different data to allow for a precise initial positioning of the robotic tool relative to the scanned object. The ultrasound device can then be advantageously be used to capture and/or track the motion or movements of the object without exposing it to unnecessary high doses of X-ray radiation. Preferably the ultrasound device may also be registered to X-ray apparatus and the robotic tool or the same frame of reference as the X-ray apparatus and the robotic tool. The X-ray apparatus may be part of the system according to the present invention or it may be part of a separate device.

In an alternative advantageous refinement the ultrasound device can be arranged so that a main detection direction of the ultrasound device extends substantially perpendicular to a central axis of a cone of operation of the robotic tool. The ultrasound device may, in other words, be arranged on a different side of the object than the robotic tool, preferably lateral to the object. This advantageously avoids contact or conflict between the robotic tool and the ultrasound device and also keeps the point or region of contact or interaction between the robotic tool and the object free for other tools and/or for observation or intervention by an operator. It is a particular advantage of the present invention that the ultrasound does not have to be positioned next to the robotic tool or even on the same side of the object as the robotic tool since it can capture the motion of the whole object or a part of the object, in particular an inner part that is not readily visible from the outside, from different directions. It can also be possible to arrange or position the ultrasound device opposite the robotic tool. The main detection direction of the ultrasound device may then extend substantially antiparallel to the central axis of the cone of operation of the robotic tool. The cone of operation of the robotic tool is defined by a substantially cone-shaped volume extending outward from a point of contact or interaction between the robotic tool and the object. The robotic tool can be moved or positioned within the cone of operation while maintaining its point of contact or interaction with the object.

In the present invention at least one ultrasound head of the ultrasound device is integrated within the robotic tool. The ultrasound head is part of a tool head that is itself part of the robotic tool or attached thereto. The ultrasound head or probe may be disposed to a side or next to the tool head. The ultrasound head does not necessarily have be enclosed by a housing of the robotic tool but may instead be attached to the robotic tool or the housing of the robotic tool from the outside. In any case, the ultrasound head is preferably rigidly connected to the robotic tool so they always move together or in tandem. This automatically and effortlessly ensures that the ultrasound device is always advantageously aligned with an axis of the robotic tool and/or always captures the motion of exactly the part of the object that is manipulated by or with the robotic tool. The ultrasound head integrated with or attached to the robotic tool may be a single or dual, in particular a 3D-ultrasound head. Depending on the situation or conditions, this allows for a compact outline of the robotic tool or a sophisticated motion capturing.

In addition to the ultrasound head integrated with the robotic tool the system comprises at least one additional ultrasound head. This additional ultrasound head is arranged independently of the robotic tool and thus advantageously provides an additional angle of motion capture.

A method according to the present invention is used for controlling a robotic medical tool. As part of the method a motion or movement of an object is captured and the robotic tool is adjusted relative to the object in dependence of the captured motion. Therein, the motion of the object is captured by means of an ultrasound device. The method according to the present invention can in particular be carried out by or with a system according to the present invention.

Advantageous refinements and embodiments of the system according to the present invention are to be regarded as advantageous refinements and embodiments of the method according to the present invention and vice versa.

Further advantages, features, and details of the present invention derive from the following description of preferred embodiments as well as from the drawing. The features and feature combinations previously mentioned in the description as well as the features and feature combinations mentioned in the following description of the figure and/or shown in the figure alone can be employed not only in the respective indicated combination but also in any other combination or taken alone without leaving the scope of the invention.

The only FIG depicts a schematic perspective view of a system according to the present invention engaged in manipulating a patient.

The only FIG shows a schematic view of an operating table 1 on which a patient 2 is positioned. Furthermore a system 3 is shown comprising a robotic tool 4, an ultrasound device 5 and a control unit 6. The control unit 6 configured to at least partially control the robotic tool 4 and the ultrasound device 5 and is for this purpose connected to the robotic tool 4 as well as to the ultrasound device 5 via respective data links or cables. The control unit 6 could alternatively be part of or integrated in the robotic tool 4 and/or the ultrasound device 5.

The robotic tool 4 presently comprises several a member or arm with segments 7. The segments 7 are connected through joints 8. The arrangement of the segments 7 and the joints 8 allows complex movements around at least three axis. The segment 7 furthest from a main body of the robotic tool may be a tool head 9 which can comprise or carry different kinds of types of tools. The tool head 9 or, more specifically a distal end of the tool head 9, is designed for manipulation of or interaction with objects. Presently the manipulated object is the patient 2 or a part thereof, in particular a region of interest (ROI) 10 of the patient. This ROI 10 may for example be or comprise a spine or a specific vertebra of the patient 2.

The ROI 10 may be considered as a point of origin of a cone of operation of the robotic tool 4. The cone of operation describes a volume of space in which the tool head 9 can move or be positioned while maintaining its contact with the ROI 10. For illustrative purposes, the cone of operation is indicated by two reference lines 11 and a central axis 12. Depending on the situation the cone of operation may not actually be cone-shaped.

It is obvious that a precise control and positioning of the tool head 9 relative to the patient 2 or the ROI 10 is essential in many applications of the robotic tool 4. For example in robot assisted spine surgery, where the robotic tool 4 supports a surgeon to place screws into a vertebra of the patient 2, the screw path must be precisely controlled to achieve the intended purpose and prevent injury of the patient 2. Therefore any motion or movement of the ROI 10 must be detected and captured. Presently this is done by means of the ultrasound device 5. At least one ultrasound head of the ultrasound device 5 is integrated with the robotic tool 4 as part of the tool head 9.

An additional ultrasound head 13 of the ultrasound device 5 is placed in contact with the patient 2 for providing an additional angle of motion capture. As can be clearly seen, the ultrasound device 5 and the ultrasound head 13 are disposed lateral to the patient 2, in particular on a different side of the patient 2 than the ROI 10 and the tool head 9. This means that a main detection direction 14 of the ultrasound device 5 extends substantially perpendicular to the central axis 12 of the cone of operation of the robotic tool 4 or the tool head 9. The ultrasound device 5 can for example detect any bone material of the patient 2, in particular in the ROI 10. Hence, the ultrasound device 5 can capture and track a motion of said bone material and by extension of the patient 2 itself.

Of course the captured motion has to be put in relation to the current position and/or motion of the tool head 9. To achieve this, the robotic tool 4 and the ultrasound device 5 can be registered to one another. Also an X-ray apparatus (not shown) can be registered to the same frame of reference too.

The motion of the patient 2 and/or the ROI 10 captured by the ultrasound device 5 - or corresponding sensor data - can be transmitted to the control unit 6. The control unit 6 processes the data received from the ultrasound device 5 and generates corresponding control signals or commands. The latter are then sent to the robotic tool 4 to position and/or move the tool head 9 accordingly. This means that the robotic tool, in particular the tool head 9, is controlled, in particular positioned and/or moved, in dependence of the motion of the patient 2 and/or the ROI 10 captured by the ultrasound device 5. The ultrasound, in other words, enables live adjustments of the robotic tool 4 during its operation.

## Claims

1. System (3) comprising a robotic medical tool (4), a motion capture device (5) and a control unit (6), wherein the motion capture device (5) is configured to capture a motion of an object (2, 10) and wherein the control unit (6) is configured to adjust the robotic tool (4) relative to the object (2, 10) in dependence of the captured motion, wherein the motion capture device (5) is an ultrasound device (5), and the robotic medical tool (4) comprises a tool head (9) arranged at a distal end of a robotic arm and designed to manipulate or interact with the object (2, 10),
wherein at least one ultrasound head of the ultrasound device (5) is integrated with the robotic medical tool (4) as part of the tool head (9) for tandem movement of this at least one ultrasound head and the tool head (9), **characterized in that**
- in addition to the at least one ultrasound head integrated with the robotic tool (4), the system (3) comprises at least one additional ultrasound head (13) arranged independently of the robotic medical tool (4) for providing an additional angle of motion capture.

2. System (3) in accordance with claim 1,
**characterised in that**
- the system (3) is configured to determine a main plane and/or direction of the motion and
- the ultrasound device (5) is arranged to capture the motion of the object (2, 10) **in that** plane and/or direction.

3. System (3) in accordance with any of the preceding claims,
**characterised in that**
the motion capture device (5) is configured to capture the motion in three dimensions.

4. System (3) in accordance with any of the preceding claims,
**characterised in that**
the ultrasound device (5) comprises at least two ultrasound heads.

5. System (3) in accordance with any of the preceding claims,
**characterised in that**
the system (3) is configured to scan the object (2, 10) and to then register the medical tool (4) to a coordinate system (3) of the scan.

6. System (3) in accordance with claim 5,
**characterised in that**
the system (3) is configured to scan the object (2, 10) with the ultrasound device (5).

7. System (3) in accordance with claim 5,
**characterised in that**
the system (3) is configured to scan the object (2, 10) with an X-ray apparatus.

8. Method for controlling a robotic medical tool (4), wherein a motion of an object (2, 10) is captured and the robotic tool (4) is adjusted relative to the object (2, 10) in dependence of the captured motion, wherein the motion of the object (2, 10) is captured by means of an ultrasound device (5),
wherein
- the motion is captured using at least one ultrasound head of the ultrasound device (5) integrated with the robotic medical tool (4) as part of a tool head (9) thereof, **characterized in that**
- this at least one ultrasound head is moved in tandem with the robotic medical tool (4) and is kept aligned with an axis of the robotic medical tool (4), and
- an additional angel of motion capture is provided by at least one additional ultrasound head (13) arranged independently of the robotic medical tool (4).

## Patentansprüche

1. System (3), umfassend ein medizinisches Roboterwerkzeug (4), eine Bewegungserfassungsvorrichtung (5) und eine Steuereinheit (6), wobei die Bewegungserfassungsvorrichtung (5) ausgestaltet ist, um eine Bewegung eines Objekts (2, 10) zu erfassen, und wobei die Steuereinheit (6) ausgestaltet ist, um das Roboterwerkzeug (4) relativ zu dem Objekt (2, 10) in Abhängigkeit von der erfassten Bewegung zu justieren, wobei die Bewegungserfassungsvorrichtung (5) eine Ultraschallvorrichtung (5) ist, und wobei das medizinische Roboterwerkzeug (4) einen Werkzeugkopf (9) umfasst, der an einem distalen Ende eines Roboterarms angeordnet ist und konzipiert ist, um das Objekt (2, 10) zu manipulieren oder mit diesem zu interagieren, wobei
mindestens ein Ultraschallkopf der Ultraschallvorrichtung (5) als Teil des Werkzeugkopfes (9) zur abgestimmten Bewegung von diesem mindestens einen Ultraschallkopf und dem Werkzeugkopf (9) in das medizinische Roboterwerkzeug (4) integriert ist, **dadurch gekennzeichnet, dass**
- das System (3) zusätzlich zu dem mindestens einen Ultraschallkopf, der in das Roboterwerkzeug (4) integriert ist, mindestens einen zusätzlichen Ultraschallkopf (13) umfasst, der unabhängig von dem medizinischen Roboterwerkzeug (4) angeordnet ist, um einen zusätzlichen Winkel der Bewegungserfassung bereitzustellen.

2. System (3) nach Anspruch 1, **dadurch gekennzeichnet, dass**
- das System (3) ausgestaltet ist, um eine Hauptebene und/oder Richtung der Bewegung zu ermitteln, und
- die Ultraschallvorrichtung (5) angeordnet ist, um die Bewegung des Objekts (2, 10) in jener Ebene und/oder Richtung zu erfassen.

3. System (3) nach einem der vorhergehenden Ansprüche, **dadurch gekennzeichnet, dass** die Bewegungserfassungsvorrichtung (5) ausgestaltet ist, um die Bewegung in drei Dimensionen zu erfassen.

4. System (3) nach einem der vorhergehenden Ansprüche, **dadurch gekennzeichnet, dass** die Ultraschallvorrichtung (5) mindestens zwei Ultraschallköpfe umfasst.

5. System (3) nach einem der vorhergehenden Ansprüche, **dadurch gekennzeichnet, dass** das System (3) ausgestaltet ist, um das Objekt (2, 10) zu scannen und dann das medizinische Werkzeug (4) in einem Koordinatensystem (3) des Scans zu registrieren.

6. System (3) nach Anspruch 5, **dadurch gekennzeichnet, dass** das System (3) ausgestaltet ist, um das Objekt (2, 10) mit der Ultraschallvorrichtung (5) zu scannen.

7. System (3) nach Anspruch 5, **dadurch gekennzeichnet, dass** das System (3) ausgestaltet ist, um das Objekt (2, 10) mit einem Röntgenapparat zu scannen.

8. Verfahren zum Steuern eines medizinischen Roboterwerkzeugs (4), wobei eine Bewegung eines Objekts (2, 10) erfasst wird und das Roboterwerkzeug (4) relativ zu dem Objekt (2, 10) in Abhängigkeit von der erfassten Bewegung justiert wird, wobei die Bewegung des Objekts (2, 10) mittels einer Ultraschallvorrichtung (5) erfasst wird, wobei
- die Bewegung unter Verwendung von mindestens einem Ultraschallkopf der Ultraschallvorrichtung (5) erfasst wird, der in das medizinische Roboterwerkzeug (4) als Teil seines Werkzeugkopfes (9) integriert ist,
**dadurch gekennzeichnet, dass**
- dieser mindestens eine Ultraschallkopf abgestimmt mit dem medizinischen Roboterwerkzeug (4) bewegt wird und mit einer Achse des medizinischen Roboterwerkzeugs (4) ausgerichtet gehalten wird, und
- ein zusätzlicher Winkel der Bewegungserfassung durch mindestens einen zusätzlichen Ultraschallkopf (13) bereitgestellt wird, der unabhängig von dem medizinischen Roboterwerkzeug (4) angeordnet ist.

## Revendications

1. Système (3), comprenant un outil (4) robotique médical, un dispositif (5) d'acquisition de mouvement et une unité (6) de commande, le dispositif (5) d'acquisition de mouvement étant configuré pour acquérir un mouvement d'un objet (2, 10) et l'unité (6) de commande étant configurée pour ajuster l'outil (4) robotique par rapport à l'objet (2, 10) en fonction du mouvement acquis, le dispositif (5) d'acquisition de mouvement étant un dispositif (5) à ultrasons et l'outil (4) robotique médical comprenant une tête (9) d'outil montée à une extrémité distale d'un bras robotique et conçue pour manipuler l'objet (2, 10) ou pour interagir avec lui,
dans lequel
- au moins une tête à ultrasons du dispositif (5) à ultrasons est intégrée à l'outil (4) robotique médical comme partie de la tête (9) de l'outil pour un déplacement en tandem de cette au moins une tête à ultrasons et de la tête (9) de l'outil,
**caractérisé en ce que**
- en plus de la au moins une tête à ultrasons intégrée à l'outil (4) robotique, le système (3) comprend au moins une tête (13) à ultrasons supplémentaire montée indépendamment de l'outil (4) robotique médical pour procurer un angle supplémentaire d'acquisition de mouvement.

2. Système (3) suivant la revendication 1,
**caractérisé en ce que**
- le système (3) est configuré pour déterminer un plan et/ou une direction principale du mouvement et
- le dispositif (5) à ultrasons est agencé pour acquérir le mouvement de l'objet (2, 10) dans ce plan et/ou cette direction.

3. Système (3) suivant l'une quelconque des revendications précédentes,
**caractérisé en ce que**
le dispositif (5) d'acquisition de mouvement est configuré pour acquérir le mouvement dans trois dimensions.

4. Système (3) suivant l'une quelconque des revendications précédentes,
**caractérisé en ce que**
le dispositif (5) à ultrasons comprend au moins deux têtes à ultrasons.

5. Système (3) suivant l'une quelconque des revendications précédentes,
**caractérisé en ce que**
le système (3) est configuré pour balayer l'objet (2, 10), puis pour enregistrer l'outil (4) médical dans un système (3) de coordonnées du balayage.

6. Système (3) suivant la revendication 5,
**caractérisé en ce que**
le système (3) est configuré pour balayer l'objet (2, 10) par le dispositif (5) à ultrasons.

7. Système (3) suivant la revendication 5,
**caractérisé en ce que**
le système (3) est configuré pour balayer l'objet (2, 10) par une installation aux rayons X.

8. Procédé de commande d'un outil (4) robotique médical, un mouvement de l'objet (2, 10) étant acquis et l'outil (4) robotique étant ajusté par rapport à l'objet (2, 10) en fonction du mouvement acquis, le mouvement de l'objet (2, 10) étant acquis au moyen d'un dispositif (5) à ultrasons,
dans lequel
- on acquiert le mouvement en utilisant au moins une tête à ultrasons du dispositif (5) à ultrasons intégrée à l'outil (4) robotique médical comme partie de sa tête (4) d'outil,
**caractérisé en ce que**
- on déplace cette au moins une tête à ultrasons en tandem avec l'outil (4) robotique médical et on la maintient alignée avec un axe de l'outil (4) robotique médical, et
- on procure un angle supplémentaire d'acquisition de mouvement par au moins une tête (13) à ultrasons supplémentaire montée indépendamment de l'outil (4) robotique médical.
